# EUROPÄISCHE PATENTANMELDUNG

(11) **EP 1 190 719 A1**
(43) Veröffentlichungstag der Anmeldung: **27.03.2002**
(21) Anmeldenummer: 01118572.5
(22) Anmeldetag: 02.08.2001
(51) Int. Cl.: A61K 47/36, A61K 9/08

(54) **Kolloidale Pharmakomodulation injizierter Arzneimittel**

(30) Priorität: 17.08.2000 DE 10040707
(71) Anmelder: B. BRAUN MELSUNGEN AG, 34212 Melsungen (DE)
(72) Erfinder: Meier, Bernd H., 64285 Darmstadt (DE); Jankowiak-Meier, Iris, 64285 Darmstadt (DE)
(74) Vertreter: Weber, Thomas, Dr. Dipl.-Chem.

(57) **Zusammenfassung**

Die Erfindung betrifft die Verwendung eines Kolloids zur Beeinflussung der molekularen Beweglichkeit, der Diffusion, der pharmakokinetischen Transferprozesse und der elektroosmotischen Eigenschaften von arzneilich wirksamen Stoffen oder Stoffgemischen im Körper.

## Beschreibung

Die Erfindung betrifft die Verwendung von kolloidosmotisch wirksamen Gemischen umfassend eine arzneilich wirksame Substanz bei der Infusion, Injektion von Arzneimitteln in den Blutkreislauf des menschlichen oder tierischen Körpers sowie einen Kit umfassend die Einzelbestandteile.

Ein Arzneistoff, der in den Blutkreislauf des Körpers injiziert wurde, wird zunächst in der zirkulierenden Blutmenge, in die er eingebracht wurde, vermischt und verdünnt. Mit dem zirkulierenden Blut wird der Arzneistoff im Körper in den Organen verteilt. In Unter anderem in Abhängigkeit von der chemischen Stabilität des Arzneistoffs, seiner Löslichkeit, seiner Molekülgröße, seiner Eignung zur Elimination und biochemischen Abbaubarkeit entfaltet nur noch ein Bruchteil der ursprünglich eingebrachten Menge des Arzneistoffs am Wirkort die gewünschte Wirkung. Sehr oft wird ein großer Teil der Arzneistoffs beispielsweise. metabolisiert und/oder eliminiert, bevor er überhaupt am Wirkort über einen hinreichend langen Zeitraum in einer relevanten Wirkkonzentration vorgelegen hat. So ist für jeden Arzneistoff, der zur Einbringung in den Blutkreislauf zugelassen ist, eine genaue Kenntnis der pharmakokinetischen Eigenschaften notwendig und vorgeschrieben .

Nach dem bisherigen Stand der Technik versucht man durch eine geeignete Steuerung der Injektionsgeschwindigkeit und Arzneimittelkonzentration sowie durch geeignete Wiederholungen Arzneistoffinjektionen den pharmakokinetischen Eigenschaften entsprechend hinreichend lange wirksame Arzneistoffkonzentrationen am Wirkort zu erzielen. Hierfür werden in der Praxis neben der wiederholten Infusion von Arzneistoffen auch Perfusoren und Injektionsmaschinen verwendet, die über längere Zeiträume kontinuierlich oder diskontinuierlich kleinere Mengen der Arzneistoffe infundieren.

Nach dem Grundsatz, dass viele Arzneistoffe giftig sind und alleine ihre Dosis entscheidend ist, wird vor allem durch die langsame und wiederholte Infusion von potentiell toxisch wirkenden Arzneistoffen versucht, gefährlich hohe Konzentrationen zu vermeiden.

Dieses Vorgehen zur Vermeidung gefährlicher Überdosierungen bedeutet jedoch eine Verzögerung des Beginns der Arzneiwirkung.
Viele hochwirksame Arzneistoffe werden deshalb in entsprechend großen Mengen von Injektionslösungen verdünnt. Hierdurch wird die Einbringung jeweils kleinerer Mengen eines Arzneistoffs technisch vereinfacht. Allen diesen Verfahren ist gemeinsam, dass durch die Mischung eine quantitative Verdünnung des Arzneistoffs im Injektionsvolumen bewirkt wird, wobei Diffusions-, Verdünnungs- und Verteilungsvorgänge im Körper, unberücksichtigt bleiben.

Ein bekanntes Standardmodell zur Beschreibung der Pharmakokinetik einer Substanz sieht direkt nach Injektionsbeginn eine Verteilung in einem zentralen Kompartiment V1 vor (Figur 1). In der Praxis entspricht die Konzentration des Arzneistoffs in diesem zentralen Kompartiment im wesentlichen der Konzentration des Arzneistoffs im Blut und näherungsweise seiner Serumkonzentration. In Abhängigkeit von dem Grad der kinetischen Ordnung (1. Ordnung, 2. Ordnung, etc.) verteilt sich der Arzneistoff von diesem zentralen Kompartiment in ein, zwei oder mehrere weitere Kompartimente V2, V3, etc. und verschwindet durch Metabolismus oder durch renale Elimination aus dem System. Für die Berechnung der aktuellen Konzentration im zentralen Kompartiment - näherungsweise die Serumkonzentration - müssen neben der Injektionsgeschwindigkeit, der Elimination oder Metabolisierung, die Größe des zentralen Kompartiments sowie die pharmakokinetischen Transferkonstanten K₁₂, K₂₁, K₁₃, K₃₁, etc. zwischen den Kompartimenten bekannt sein.
Für die mit dem zentralen Kompartiment in Verbindung stehenden weiteren Kompartimente gibt es keine physiologisch präzise Entsprechung. Maitre und Schafer (Anaesthiology 1990, 73, 332-336) haben für die zur Kalkulation der Konzentration eines bestimmten Arzneistoffs im zentralen Kompartiment notwendigen Variablen in Abhängigkeit von Gewicht, Alter und Geschlecht präzise Algorithmen publiziert.

Aufgabe der Erfindung ist es, körperliche Parameter, die einen wesentlichen Einfluss auf die Pharmakokinetik eines in Körpergefäße eingebrachten arzneilich wirksamen Stoffs haben, steuerbar zu beeinflussen. Durch geeignete Maßnahmen soll also auf diese, von der Arzneistoffapplikation primär unabhängigen Parameter, Einfluss genommen werden.

Das der Erfindung zugrunde liegende Problem wurde gelöst durch die Verwendung von gut steuerbaren kolloidalen Gemischen, die ein hinreichend großes Molekulargewicht aufweisen, die aufgrund ihrer kurzen Halbwertszeit bereits nach kurzer Zeit wieder ausgeschieden werden, die auch bei längerer und wiederholter Anwendung nicht in den Organen, insbesondere des retikuloendothelialen Systems gespeichert werden und auf die aufgrund ihrer ihrer polaren Substitution elektroskopischen Effekte wirken können.

Eigene Untersuchungen haben ergeben, dass physiologische Windkesselfunktionen des rechten Vorhofs sowie die Mischbarkeit des Blutes und Diffusion durch hohe Kolloidkonzentrationen deutlich vermindert werden.

Bei vergleichenden Untersuchungen zur Messung des Herzzeitvolumens mit der Thermodilutionsmethode, einem neuen Verfahren der transcardialen Impedanzmessung und dem Verfahren nach Fick wurde beobachtet, dass es nach der Infusion großer Kolloidmengen mit dem Thermodilutionsverfahren zu signifikanten Abweichungen der ermittelten Herzzeitvolumina im Vergleich zu den anderen Verfahren kam.

Diese Abweichung war bei Verwendung von Kolloiden mit einem mittleren Molekulargewicht um 450.000 deutlich größer als bei Kolloiden mit mittleren Molekulargewichten um 200.000 und kleiner.
In vitro Untersuchungen zeigen, dass die Dilution eines Indikators durch die Anwesenheit eines Kolloids im Messvolumen konzentrationsabhängig verlangsamt wird.
Es ist bekannt, dass die von Brown 1827 erstmalig in wässrigen Lösungen mikroskopisch beobachtete Molekularbewegung mit steigendem Molekulargewicht eines gelösten Kolloids deutlich abnimmt. Das bedeutet, dass die Dilution, aber auch die Diffusion in solchen wässrigen Lösungen vermindert ist.

Auf der Grundlage der durchgeführten Experimente und den Erkenntnissen am Versuchstier wurde gefunden, dass sich das pharmakokinetische Schicksal und die Eigenschaften von injizierten Stoffen durch Beimischung von Kolloiden unter physiologischen Bedingungen relevant beeinflussen lässt.
Daraus folgt, dass der Effekt damit konzentrationsabhängig auf die Pharmakokinetik von Arzneistoffen, denen Kolloide beigemischt wurden, insbesondere auf die pharmakokinetischen Transferkonstanten (K₁₂, K₂₁, K₁₃, K₃₁) wirkt und diese verändert.
Zusätzlich wird durch die Infusion von als Plasmaexpander wirksamen Kolloidlösungen eine Vergrößerung der zirkulierenden Blutmenge bewirkt, womit eine Vergrößerung des zentralen Kompartiments V1 verbunden ist.

Ein spezifisch kolloidaler Effekt, der zur Beeinflussung des Schicksals arzneilich wirksamer Stoffe beiträgt, sind elektroosmotische Kräfte, die von der Oberfläche von mit polaren Substituenten besetzen Makromolekülen ausgehen.

Durch polare Substituenten können elektrische Ladungsmuster in die kolloidale Oberflächenstruktur eingeführt werden. Durch Anschluss an elektrische Felder werden Bewegungen der geladenen Oberfläche relativ zu der benachbarten flüssigen Phase ausgelöst (Elektroosmose). Diese relative Bewegungen können beispielsweise durch Elektrophorese sichtbar gemacht werden.

Die Substitution durch Einbindung von organischen Säuren in das Stärkemolekül mittels Esterbindung ist ein in der Kolloidchemie bekannter Syntheseweg. Durch Veresterung werden u.a. polare Substituenten in die Oberfläche des Stärkemoleküls eingeführt.
So können durch die Veresterung von Stärke mit der Aminosäure Cystein als Cysteylstärke-Ester entsprechend polare Strukturen in die Oberfläche eingeführt werden. Auch die Effekte von so aufbaubaren Disulfidbrücken können hierdurch genutzt werden. Veresterungen mit Monoamino-Dicarbonsäuren, z.B., mit den Aminosäuren Asparagin und Glutamin oder mit Diamino-Monocarbonsäuren Arginin, Lysin und Histidin bilden ebenfalls entsprechend polare Oberflächenstrukturen auf der Stärke aus.

Auch die Synthese von Polymeren der Stärke, die in kovalenter Bindung polare Substituenten, Verbindungen mit -S-H oder anderen Gruppen, beispielsweise Fluoresceinisothiocyanat tragen, können zur Erhöhung der Polarität der Stärke beitragen. De Beider und Granath (Carbohydr. Research, 1973, 30, 375-378) haben 1973 die Einführung von Fluoresceinisothiocyanat in Dextrane und Stärke (FITC-HES) beschrieben. Nach diesem Verfahren von den Erfindern hergestellte Präparationen von FITC-markierten HES wurden nach Reinigung in einem Gegenstrom-Dialyse-Verfahren in Wistar-Ratten infundiert und gut vertragen. Auch Untersuchungen mit einer niedrig substituierten FITC-HES (DS=0,02) zeigten, abgesehen von spezifischen Speicherphänomenen, keinerlei histopathologische Organbefunde (vgl. Förster und Meier (Infusionstherapie, Infusionsmedizin, 1999, 26, 45-52)). In vorteilhafter Ausgestaltung der Erfindung können dergestalt gebildete polare Oberflächenstrukturen der Stärke durch Elektrophorese getrennt bzw. isoliert werden.

Durch Versuche der Erfinder konnte festgestellt werden, dass im Körper lokal abgrenzbare elektrische Wirkungen auf injizierte Arzneistoffe ausgeübt werden können. Die Figur 2 zeigt den Versuchsaufbau eines elektrophysiologischen Selbstversuchs. Mit einem transcardialen Impedanz-Messgerät, wie in DE-A-19533663 beschrieben, wird ein hochfrequenter Wechselstrom zwischen internen Katheterelektroden und externen, auf der Haut über dem Herzen angebrachten Hautelektroden im wesentlichen durch das Herz geführt. Die Figur 3 zeigt den Verlauf einer transcardialen Impedanzmesskurve (I = Zeitpunkt der Injektion, Aufzeichnungsgeschwindigkeit = 25 mm/s) bei Injektion einer polaren arzneilich wirksamen Substanz (Ascorbinsäure) in eine Handvene. Nachdem die Substanz das transcardiale Messfeld erreicht hat, tritt eine deutliche Veränderung der elektrischen Impedanz im Messfeld auf. Vergleichbare Phänomene sind auch bei Verwendung von Gleichstrom messbar. Es ist ersichtlich, dass durch eine entsprechende anatomisch-topografische Platzierung von Katheterelektroden örtlich definierte und begrenzte elektrische Felder aufgebaut werden können, wobei durch eine injizierte arzneilich wirksame Substanz elektrischer Polarität, die durch die Blutzirkulation in dieses elektrische Feld hineingetragen wird, ein unter physiologischen Bedingungen relevanter messbarer Strom fließt. Dieser Effekt wird in Figur 3 durch die Widerstandsänderung des zwischen den Stromelektroden messbaren Stroms belegt.

Für einen schwachen Gleichstrom kann bei bekannter Dielektrizität, Viskosität, Zeta-Konstante des Injektates und bekanntem eingestellten Potentialgradienten das Ausmaß eines infundierten elektroosmotisch wirksamen Prinzips berechnet werden (Physikalische Pharmazie, Alfred N. Martin & James S. Swarbrick, Wissenschaftliche Verlagsgesellschaft mbH, Stuttgart, 1980, Seite 453).

In Verbindung mit Stromgeneratoren und Anschlusselektroden am oder im Körper lassen sich für polare - polar substituierte - Kolloide elektroosmotische Effekte in wählbaren, von der Elektrodenlage abhängigen anatomischen Strukturen zur Wirkung bringen. Dies bedeutet die Möglichkeit, arzneilich wirksame Substanzen in Verbindung mit geeigneten elektroosmotischen, polaren Kolloiden in abgrenzbaren anatomischen Strukturen zu beeinflussen. Beispielsweise können Adhäsions- und Coating-Effekte des Kolloids für den arzneilich wirksamen Stoff an Körperstrukturen beeinflusst werden.

Auch die Mischungs- und Diffusionseigenschaften und Partialdrücke von physikalisch gelösten Gasen (hier als arzneilich wirksame Substanz) können durch entsprechende Kolloide mit polarer Oberflächenstruktur beeinflusst werden.

Die im vorigen beschriebene kolloidale Einfluss auf die Brown'sche Molekularbewegung, die Dilutions- und Diffusionsbedingungen, die pharmakokinetischen Transferprozesse, die Verteilung und Elektroosmose von arzneilich wirksamen Stoffen soll im folgenden als kolloidale Pharmakomodulation zusammengefasst werden.

Im einzelnen betrifft die Erfindung den durch die Patentansprüche definierten Gegenstand, auf den hiermit ausdrücklich Bezug genommen wird.

Prinzipiell kommen für die erfindungsgemäße Beeinflussung des Schicksals arzneilich wirksamer Stoffe nach Einbringung in den Körper im Hinblick auf ihre Brown sche Molekularbewegung, pharmakokinetische Transferprozesse, Verteilung und Elektroosmose, alle Kolloide in Betracht, die
- geeignet sind, in den Körper eingebracht zu werden,
- aufgrund eines hinreichend großen Molekulargewichts einen Effekt auf die Brown'sche molekulare Beweglichkeit haben,
- verhältnismäßig unabhängig von ihrem Molekulargewicht aus dem Körper (renal) eliminiert werden können,
- mit der zur Einbringung in den Körper vorgesehenen arzneilich wirksamen Substanz gemischt werden oder gemeinsam in den Körper eingebracht werden können,
- deren Konzentration durch Infusionen vor und nach der Injektion der arzneilich wirksamen Substanz beeinflusst bzw. gesteuert werden kann,
- die bei wiederholter AnwendungNerabreichung im Körper nicht kumulieren,
- deren Infusion zu einer steuerbaren Expansion des zentralen Kompartiments führt,
- zu einer Erhöhung der Blutviskosität und zu einer Verbesserung der Mikrozirkulation für den arzneilich wirksamen Stoff führen,
- eine Aufnahme oder Phagozytose des arzneilich wirksamen Stoffes in zirkulierende und sessile Zellen des Retikuloendothelialen Systems einschränken,
- zu einer Verminderung der Fließgeschwindigkeit in Infusionssystemen führen (Steuerbarkeit der Infusionsgeschwindigkeit),
- zu einer steuerbaren Veränderung von Partialdrucken verschiedener, im Blut gelöster Gase führen können,
- zu Veränderungen der elektrischen Ladungsstrukturen im Blut führen, mit denen sich die molekularen Assoziationen von in den Körper eingebrachten arzneilich wirksamen Stoffen beeinflussen lassen,
- nach Infusion aufgrund ihrer polaren Oberflächenstruktur mit elektrischen Strömen bzw. Feldern elektroosmotisch beeinflusst werden können.

Erfindungsgemäß handelt es sich vorzugsweise um Kolloide, deren Wasserlöslichkeit durch Einfluss eines polar wirkenden Substituenten verbessert und deren enzymatische Spaltung durch diese Substituenten deutlich verzögert wird, wobei deren elektroosmotische Wirkung durch polare Substitution erhöht wird.

Die kolloidbildenden Makromoleküle sind ausgewählt aus der Gruppe bestehend aus Polysacchariden, modifizierten Polysacchariden, Polypeptiden, modifizierten Polypeptiden und Eiweißen, wie etwa Albuminen.

Bei den Polysacchariden handelt es sich vorzugsweise um Cellulose, Stärke und Dextran, wobei als Polysaccharid Hydroxyethylstärke (HES) bevorzugt ist. Da die Brown'sche Molekularbewegung in Flüssigkeiten durch größere Moleküle mehr eingeschränkt wird als durch kleinere, weisen die erfindungsgemäß eingesetzten Hydroxyethylstärken vorzugsweise ein im Verhältnis zum Substitutionsgrad DS höheres Molekulargewicht auf. Gemäß der Erfindung kommen insbesondere solche Hydroxyethylstärken in Betracht, welche einen Substitutionsgrad (Degree of Substitution) DS < 0,4 aufweisen. Der Substitutionsgrad DS ist definiert als der Anteil der substitutierten Anhydroglucose-Einheiten aller Anhydroglucose-Einheiten. Ihn kann man bestimmen aus der Menge der unsubstituierten Glucose nach Hydrolyse einer Probe. Vorzugsweise beträgt der Substitutionsgrad DE wenigstens 0,15, besonders bevorzugt 0,3. Die erfindungsgemäß eingesetzten Hydroxyethylstärken weisen vorzugsweise ein mittleres Molekulargewicht von über 100.000 auf, ganz besonders bevorzugt ein mittleres Molekulargewicht über 250.000 auf.
Andere erfindungsgemäße kolloidale Polysaccharide sind Ester der Stärke mit der Aminosäure Cystein als Cysteylstärke, und/oder Ester mit Monoamino-C₃-C₁₀-Carbonsäuren, vorzugsweise Monoamino-C₃-C6-Carbonsäuren, insbesondere mit den Aminosäuren Asparagin und Glutamin oder als Ester mit Diamino-C₂-C₁₀-Monocarbonsäuren, vorzugsweise den Diamino-C₂-C₆-Monocarbonsäuren, insbesondere mit den Aminosäuren Arginin, Lysin und Histidin. Die so substituierten Stärkeverbindungen weisen einen Substitutionsgrad DS von < 0,4, vorzugsweise <0,2 auf.

Alternativ zum modifizierten Polysaccharid Hydroxyethylstärke [Poly(O-Hydroxyethylstärke] oder in Kombination damit kann Carboxy-C₁-C₁₀-alkyl-Stärke, vorzugsweise kann Carboxy-C₁-C₅-alkyl-Stärke eingesetzt werden. Die CarboxyalkylStärke weist einen Substitutionsgrad von 0,1 bis < 0,4, vorzugsweise einen Substitutionsgrad von < 0,2 auf

Die erfindungsgemäß als kolloidosmotisch wirksame Makromoleküle eingesetzten Polypeptide und modifizierten Polypeptide sind ausgewählt aus der Gruppe bestehend aus Gelatine, Oxypolygelatine und Gelatinesuccinat. Die Eiweiße sind vorzugsweise Albumin, menschliches Albumin, Spaltprodukte des Albumins oder rekombinantes menschliches Serum-Albumin (rHSA).

Im Gegensatz zu Kolloiden, die als Plasmaexpander eingesetzt werden, ist gemäß der Erfindung eine unsteuerbare anhaltende Wirkung auf den kolloidosmotischen Druck bzw. ein längeres Verbleiben im Blut als zusätzlich pharmakologische Wirkung nicht erwünscht. Für den erfindungsgemäß beabsichtigten Effekt ist lediglich ein Verbleiben im Kreislauf für den Zeitraum erwünscht, in dem sich der kolloidal zu beeinflussende arzneilich wirksame Stoff selbst in dem zentralen Kompartiment befinden soll. Idealerweise werden die Substanzen danach also während einiger Nierenpassagen wieder aus dem Körper eliminiert. Es ist bekannt, dass die Serumhalbwertszeit von Hydroxyethylstärke in erster Linie von ihrem Substitutionsgrad DS abhängt. Das Molekulargewicht spielt hierbei eine untergeordnete Rolle, ist jedoch für die kolloidale Wirkung notwendig. Für die Wasserlöslichkeit und die erwünschten elektroosmotischen Effekte der Stärke ist jedoch eine Mindestmenge von Hydroxyethylgruppen notwendig. Eine niedrig substituierte HES mit einem Substitutionsgrad unter 0,4, zum Beispiel von 0,3, wäre bei hinreichend größeren Molekulargewichten über 100.000, beispielsweise 250.000, zur kolloidalen Modulation von arzneilich wirksamen Stoffen befriedigend wirksam, würde jedoch der Aufenthaltszeit arzneilich wirksamer Stoffe im zentralen Kompartiment entsprechen. Eine gewünschte verlängerte Aufrechterhalten einer bestimmten Kolloidkonzentration im Blut könnte durch weitere Kolloidinfusion erreicht werden.

Wie bei den durchgeführten Experimenten soll die Konzentration des Kolloids zwischen 2 bis 25 Gew.-%, vorzugsweise 3 bis 10 Gew.-% der wässrigen Lösung betragen.

Erfindungsgemäß geeignete Kolloide können, wie aus dem Stand der Technik bekannt ist, aus polydispersen Kolloidgemischen mittels Elektrophorese, Chromatographie oder Filtration isoliert werden.

Die erfindungsgemäß zur Anwendung kommenden injizierbaren wässrigen Lösungen eines Arzneimittels weisen eine Osmolarität zwischen 250 und 400 mOsmol/l, vorzugsweise zwischen 300 und 350 mOsmol/l auf. Die Einstellung der Tonizität der zu injizierenden Lösung kann durch einen zusätzlichen Kationenanteil von 100 - 170 mmol/l, vorzugsweise 100 - 150 mmol/l, und einen Anionenanteil von 100 bis 140 mmol/l, vorzugsweise 100 - 150 mmol/l vorgenommen werden. Ein Anteil der Kationen- und/oder Anionenkonzentration kann durch ein natürliches oder synthetisches Polyol ersetzt sein. Zu diesem Zweck geeignete Polyole sind im Stand der Technik bekannt. Beispielhaft seien in diesem Zusammenhang Zucker wie Glucose und synthetische Polyole wie Sorbitol oder Xylitol genannt. Bei den erfindungsgemäß verwendeten Zusammensetzungen handelt es sich um in den Blutkreislauf injizierte Kolloidlösungen und nicht um Suspensionen, Emulsionen oder Gele.

Der pH-Wert der zur Anwendung kommenden Lösung ist im wesentlichen bestimmt von den an die Stabilität des zu verabreichenden Arzneimittels in der wässrigen Lösung geknüpften Erfordernissen. Der pH-Wert kann zwischen 1,5 und 12, vorzugsweise zwischen 4,5 und 8, liegen. Die erfindungsgemäß verwendeten Lösungen sind partikelfrei und frei von emulsionsbildenden perfluorierten organischen Verbindungen. Bei den erfindungsgemäß verwendeten Zusammensetzungen handelt es sich also weder um Emulsionen noch um Suspensionen. Die injizierbaren Zusammensetzungen können ferner in der Infusionstherapie übliche und physiologisch verträgliche Zusatzstoffe und Hilfsstoffe enthalten, wie beispielsweise Puffersysteme. Als Puffer kann der Lösung die Aminosäure Histidin, vorzugsweise in einer Dosierung von 50 - 150 mmol/l und Histidinhydrochlorid, vorzugsweise in einer Dosierung von 5 - 20 mmol/l zugesetzt werden.

Erfindungsgemäß können als arzneilich wirksamer Bestandteil Arzneimittel eingesetzt werden, die ausgewählt sind aus der Gruppe bestehend aus Abmagerungsmittel/Appetitzügler, Acidosetherapeutika, Aminosäuren (z. B. Histidin) oder modifizierte Aminosäuren, Analeptika/Antihypoxämika, Analgetika/Antirheumatika, Anthelminthika, Antiallergika, Antianämika, Antiarrhythmika, Antibiotika/Antiinfektiva, Antidementiva (Nootropika), Antidiabetika, Antidota, Antiemetika/Antivertiginosa, Antiepileptika, Antihämorrhagika (Antifibrinolytika u. andere Hämostatika), Antihypertonika, Antihypoglykämika, Antihypotonika, Antikoagulantia, Antimykotika, Antiparasitäre Mittel (intern), Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Cholagoga und Gallenwegstherapeutika, Cholinergika, Corticoide (intern), Dermatika (intern), Diätetika/Ernährungstherapeutika, Diagnostika und Mittel zur Diagnosevorbereitung, Diuretika, Durchblutungsfördemde Mittel, Entwöhnungsmittel, Enzyminhibitoren, Enzympräparate und Transportproteine, Fibrinolytika, Geriatrika, Gichtmittel, Grippemittel, Gynäkologika, Hämorrhoidenmittel (Proktologika), Hepatika, Hypnotika/Sedativa, Hypophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschildddrüsenhormone/Calciumstoflwechselregulatoren/Oste-oporosemittel, Neuropathiepräparate und andere neurotrope Mittel, Neurotransmitter (z. B. Dopamin) oder modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, Immunglobuline und Impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer. Der Arzneimittelwirkstoff kann in der injizierbaren Lösung in einem Anteil von 0,5 von 25 Gew.-% vorzugsweise von 2 bis 15 Gew.-%, besonders bevorzugt 5 bis 10 Gew.-% enthalten sein, bezogen auf die Gesamtmenge der injizierbaren Lösung.

Die Erfindung betrifft ferner einen Kit umfassend wenigstens einen arzneilich wirksamen Bestandteil sowie eine kolloidale Substanz, insbesondere aus der Gruppe der Polysaccharide, modifizierten Polysaccharide und Gelatine.

Die Herstellung der injizierbaren wässrigen Arzneimittellösung kann nach bekannten Methoden des Standes der Technik erfolgen, insbesondere durch Mischen der Einzelkomponenten unter Bildung der Lösung oder durch gemeinsame bzw. zeitversetzte Infusion. Zur Herstellung des Kits werden die Einzelkomponenten in geeignete Behältnisse gefüllt, verschlossen und getrennt voneinander in Form des Kits bis zur Anwendung zur Verfügung gestellt. Gemäß der Erfindung können auf besondere arzneilich wirksame Stoffe oder spezielle Wirkprofile von arzneilich wirksamen Stoffen abgestimmte Kolloide beispielsweise durch Elektrophorese oder andere chromatographische Verfahren bestimmt, isoliert und für die kolloidale Modulation des Arzneistoffs bereitgestellt werden. Erfindungsgemäß kann durch Stromgeneratoren und mindestens 2 Stromelektroden, die selektiv an anatomische Strukturen angelegt werden können, die als Leitungspfad zur Einbringung einer wählbar großen elektrischen Energie in einen von Gefäßen durchströmten Bereich des Körpers dienen, wobei auf die in Gefäße eingebrachten Gemische, bestehend aus mindestens einem arzneilich wirksamen Stoff und einem zusätzlich eingebrachten polar substituierten Kolloid, eine auf diesen Bereich beschränkte elektroosmotische Energie wirkt.

Besonders vorteilhaft ist die Möglichkeit, die örtliche Beschränkung dieser Stromwirkungen durch die Impedanzmessungen auch an anderen Körperregionen zu überprüfen.

Durch die Erfindung kann die Wirkung, Pharmakokinetik, und Verträglichkeit von arzneilich wirksamen Stoffen nach deren Einbringung in den Körper beeinflusst und verbessert werden.

## Patentansprüche

1. Verwendung eines Kolloids zur Herstellung einer wässrigen injizierbaren Verwendung (eines Arzneimittels) umfassend wenigstens einen arzneilich wirksamen Bestandteil zur Beeinflussung der molekularen Beweglichkeit, der Diffusion, der pharmakokinetischen Transferprozesse sowie elektrophoretischen Eigenschaften des arzneilich wirksamen Bestandteils.

2. Verwendung nach Anspruch 1, **dadurch gekennzeichnet, dass** das Kolloid vor, gleichzeitig oder nach der Infusion des arzneilich wirksamen Bestandteils in den menschlichen oder tierischen Körper erfolgt.

3. Verwendung eines infundierbaren Kolloids zur kolloidalen Pharmakomodulation wenigstens eines in den menschlichen oder tierischen Körper eingebrachten arzneilich wirksamen Stoffs oder Stoffgemisches.

4. Verwendung nach irgendeinem der Ansprüche 1, 2 oder 3, **dadurch gekennzeichnet, dass** die kolloidale Substanz ein Kolloide bildendes Makromolekül ist.

5. Verwendung nach Anspruch 4, **dadurch gekennzeichnet, dass** das Makromolekül ausgewählt ist aus der Gruppe bestehend aus Polysacchariden, modifizierten Polysacchariden, Peptiden, modifizierten Peptiden und Eiweißen.

6. Verwendung nach Anspruch 5, **dadurch gekennzeichnet, dass** das Polysaccharid ausgewählt ist aus der Gruppe bestehend aus Cellulose, Stärke, Dextran oder deren modifizierten Derivaten.

7. Verwendung nach Anspruch 5 oder 6, **dadurch gekennzeichnet, dass** das modifizierte Polysaccharid Hydroxyethylstärke [Poly(O-Hydroxyethylstärke], Carboxy-C₁-C₁₀-alkyl-Stärke oder ein Gemisch aus Hydroxyethylstärke und Carboxy-C₁-C₁₀-alkyl-Stärke ist.

8. Verwendung nach Anspruch 7, **dadurch gekennzeichnet, dass** die Hydroxyethylstärke einen Substitutionsgrad DS < 0,4 aufweist.

9. Verwendung nach Anspruch 7, **dadurch gekennzeichnet, dass** die Hydroxyethylstärke ein mittleres Molekulargewicht über 100.000 aufweist.

10. Verwendung nach Anspruch 9, **dadurch gekennzeichnet, dass** die Hydroxyethylstärke ein mittleres Molekulargewicht über 250.000 aufweist.

11. Verwendung nach Anspruch 8, **dadurch gekennzeichnet, dass** die Hydroxyethylstärke einen Substitutionsgrad DS < 0,4 und ein Molekulargewicht über 100.000 aufweist.

12. Verwendung nach Anspruch 11, **dadurch gekennzeichnet, dass** die Hydroxyethylstärke einen Substitutionsgrad DS < 0,4 und ein Molekulargewicht über 250.000 aufweist.

13. Verwendung nach Anspruch 12, **dadurch gekennzeichnet, dass** die Hydroxyethylstärke einen Substitutionsgrad DS < 0,3 aufweist.

14. Verwendung nach Anspruch 13, **dadurch gekennzeichnet, dass** die Hydroxyethylstärke einen Substitutionsgrad DS < 0,3 und ein Molekulargewicht unter 70.000 aufweist.

15. Verwendung nach Anspruch 6, **dadurch gekennzeichnet, dass** die Hydroxyethylstärke, das Dextran und die Cellulose zusätzliche, über Estergruppen gebundene polare Gruppen aufweist.

16. Verwendung nach Anspruch 15, **dadurch gekennzeichnet, dass** sich die polare Gruppe aus der Gruppe bestehend aus Cystein, einer Monoamin-C₃-C₁₀-Dicarbonsäure, Glutamin, Asparagin, einer Diamino-C₂-C₁₀-monocarbonsäure, Lysin, Arginin und Histidin ableitet.

17. Verwendung nach Anspruch 7, **dadurch gekennzeichnet, dass** die Carboxyalkylstärke ausgewählt ist aus der Gruppe bestehend aus Carboxymethylstärke, Carboxyethylstärke und Carboxypropylstärke.

18. Verwendung nach Anspruch 7 oder 17, **dadurch gekennzeichnet, dass** die Carboxyalkylstärke einen Substitutionsgrad von 0,1 bis < 0,4 aufweist.

19. Verwendung nach Anspruch 18, **dadurch gekennzeichnet, dass** die Carboxyalkylstärke einen Substitutionsgrad von < 0,2 aufweist.

20. Verwendung nach Anspruch 5, **dadurch gekennzeichnet, dass** die Peptide ausgewählt sind aus der Gruppe bestehend aus Gelatine, Oxypolygelatine, Gelatinesuccinat.

21. Verwendung nach Anspruch 5, **dadurch gekennzeichnet, dass** die Eiweiße ausgewählt sind aus der Gruppe bestehend aus Albumin, menschlichem Albumin, Spaltprodukten des Albumins und rekombinantem menschlichem Serum-Albumin.

22. Verwendung nach irgendeinem der vorangehenden Ansprüche, **dadurch gekennzeichnet, dass** die Konzentration des Kolloids 2 bis 25 Gew.-%, bezogen auf die Gesamtzusammensetzung, ist.

23. Verwendung nach irgendeinem der vorangehenden Ansprüche, **dadurch gekennzeichnet, dass** der arzneilich wirksame Bestandteil ausgewählt ist aus der Gruppe bestehend aus Abmagerungsmittel/Appetitzügler, Acidosetherapeutika, Aminosäuren (z. B. Histidin) oder modifizierte Aminosäuren, Analeptika/Antihypoxämika, Analgetika/Antirheumatika, Anthelminthika, Antiallergika, Antianämika, Antiarrhythmika, Antibiotika/Antiinfektiva, Antidementiva (Nootropika), Antidiabetika, Antidota, Antiemetika/Antivertiginosa, Antiepileptika, Antihämorrhagika (Antifibrinolytika u. andere Hämostatika), Antihypertonika, Antihypoglykämika, Antihypotonika, Antikoagulantia, Antimykotika, Antiparasitäre Mittel (intern), Antiphlogistika, Antitussiva/Expektorantia, Arteriosklerosemittel, Balneotherapeutika und Mittel zur Wärmetherapie, Betarezeptoren-, Calciumkanalblocker und Hemmstoffe des Renin-Angiotensin-Systems, Broncholytika/Antiasthmatika, Cholagoga und Gallenwegstherapeutika, Cholinergika, Corticoide (intern), Dermatika (intern), Diätetika/Ernährungstherapeutika, Diagnostika und Mittel zur Diagnosevorbereitung, Diuretika, Durchblutungsfördernde Mittel, Entwöhnungsmittel, Enzyminhibitoren, Enzympräparate und Transportproteine, Fibrinolytika, Geriatrika, Gichtmittel, Grippemittel, Gynäkologika, Hämorrhoidenmittel (Proktologika), Hepatika, Hypnotika/Sedativa, Hypophysen-, Hypothalamushormone, regulatorische Peptide und ihre Hemmstoffe, Immuntherapeutika und Zytokine, Infusions- und Standardinjektionslösungen, Organperfusionslösungen, Kardiaka, Karies-, Parodontosemittel und andere Dentalpräparate, Koronarmittel, Laxantia, Lipidsenker, Neuraltherapeutika, Magen-Darm-Mittel, Migränemittel, Mineralstoffpräparate, Muskelrelaxantia, Narkosemittel, Nebenschildddrüsenhormone/Calciumstoffwechselregulatoren/Oste-oporosemittel, Neuropathiepräparate und andere neurotrope Mittel, Neurotransmitter (z. B. Dopamin) oder modifizierte Neurotransmitter, Ophthalmika, Otologika, Parkinsonmittel und andere Mittel gegen extrapyramidale Störungen, Psychopharmaka, Sinusitismittel, Roborantia/Tonika, Schilddrüsentherapeutika, Sera, Immunglobuline und Impfstoffe, Sexualhormone und ihre Hemmstoffe, Spasmolytika, Thrombozytenaggregationshemmer, Tuberkulosemittel, Umstimmungsmittel, Urologika, Venentherapeutika, Vitamine, Wundbehandlungsmittel, Zytostatika und Metastasenhemmer.

24. Verwendung nach irgendeinem der vorangehenden Ansprüche, **dadurch gekennzeichnet, dass** die Konzentration des arzneilich wirksamen Bestandteils 0,5 bis 25 Gew-%, bezogen auf die Gesamtmenge der injizierbaren Zusammensetzung ist.

25. Verwendung nach irgendeinem der vorangehenden Ansprüche, **dadurch gekennzeichnet, dass** sie eine Osmolarität zwischen 250 und 400 mOsmol/l aufweist.

26. Verwendung nach irgendeinem der vorangehenden Ansprüche, **dadurch gekennzeichnet, dass** die Kolloide erhältlich sind durch Elektrophorese, Flitration oder Chromatographie.

27. Kit umfassend, räumlich getrennt voneinander, jeweils in wässriger Lösung, ein injizierbares Kolloid und einen arzneilich wirksamen Bestandteil, wie in irgendeinem der Ansprüche 1 bis 26 definiert.
